# EUROPÄISCHE PATENTSCHRIFT

(11) **EP 2 331 156 B1**
(45) Veröffentlichungstag und Bekanntmachung des Hinweises auf die Patenterteilung: **29.02.2012**
(21) Anmeldenummer: 09778051.4
(22) Anmeldetag: 22.08.2009
(51) Int. Cl.: A61L 31/10, C08G 18/44, C08G 64/02, C09D 175/08

(54) **TCD-BASIERTE HYDROPHILE POLYURETHANLÖSUNGEN**
TCD BASED HYDROPHILIC POLYURETHANE DISPERSIONS
SOLUTIONS DE POLYURÉTHANE HYDROPHILES À BASE DE TCD

(30) Priorität: 04.09.2008 EP 08015574
(43) Veröffentlichungstag der Anmeldung: 15.06.2011
(73) Patentinhaber: Bayer MaterialScience AG, 51368 Leverkusen (DE)
(72) Erfinder: KÖCHER, Jürgen, 40764 Langenfeld (DE); WAMPRECHT, Christian, 41472 Neuss (DE)
(86) Internationale Anmeldenummer: PCT/EP2009/006102
(87) Internationale Veröffentlichungsnummer: WO 2010/025841

(56) Entgegenhaltungen:
- WO-A-2006/093355
- WO-A-2006/109816
- US-A- 5 962 620

## Beschreibung

Die vorliegende Erfindung betrifft neuartige Polyurethanharnstofflösungen, welche zur Herstellung von hydrophilen Beschichtungen auf verschiedensten Substraten verwendet werden können.

Insbesondere im medizinischen Bereich sind hydrophile Beschichtungen von Oberflächen medizintechnischer Geräte von Bedeutung, da ihre Benutzung dadurch stark verbessert werden kann. Das Einsetzen und Verschieben von Urin- oder Blutgefäßkathetern wird dadurch einfacher, dass hydrophile Oberflächen im Kontakt mit Blut oder Urin einen Wasserfilm adsorbieren. Hierdurch wird die Reibung der Katheteroberfläche gegenüber den Gefäßwänden verringert, so dass sich der Katheter leichter einsetzen und bewegen lässt. Auch eine direkte Wässerung der Geräte vor dem Eingriff kann vorgenommen werden, um durch die Bildung eines homogenen Wasserfilms die Reibung zu vermindern. Die betroffenen Patienten haben weniger Schmerzen, und das Risiko von Verletzungen der Gefäßwände wird dadurch reduziert. Darüber hinaus besteht bei der Anwendung von Kathetern immer die Gefahr, dass sich Blutgerinnsel bilden. In diesem Kontext werden im Allgemeinen hydrophile Beschichtungen als hilfreich für antithrombogene Beschichtungen angesehen.

Zur Herstellung von entsprechenden Oberflächen eignen sich grundsätzlich Polyurethanbeschichtungen, die ausgehend von Lösungen oder Dispersionen entsprechender Polyurethane hergestellt werden.

So beschreibt die US 5,589,563 die Verwendung von Beschichtungen mit oberflächenmodifizierten Endgruppen für im Bereich der Biomedizin verwendete Polymere, welche auch zur Beschichtung von medizinischen Geräten verwendet werden können. Die resultierenden Beschichtungen werden ausgehend von Lösungen oder Dispersionen hergestellt und die polymeren Beschichtungen umfassen unterschiedliche Endgruppen, welche ausgewählt werden aus Aminen, fluorierten Alkanolen, Polydimethylsiloxanen und aminterminierten Polyethylenoxiden. Diese Polymere weisen jedoch als Beschichtung für medizinische Geräte keine zufriedenstellenden Eigenschaften, insbesondere im Hinblick auf die erforderliche Hydrophilie, auf.

Ein Nachteil von wässrigen Dispersionen, wie sie unter anderem in der US 5,589,563 beschrieben sind, ist darüber hinaus, dass durch die Größe der dispergierten Partikel die Beschichtungen relativ rau sind. Darüber hinaus sind die resultierenden Beschichtungen aus wässrigen Dispersionen im Allgemeinen nicht ausreichend stabil ausgebildet. Daher gibt es einen Bedarf hinsichtlich von hydrophile Beschichtungssystemen, welche eine hervorragende Hydrophilie und gleichzeitig eine relativ glatte Oberfläche und eine hohe Stabilität aufweisen.

Polyurethanlösungen an sich sind aus dem Stand der Technik bekannt, wurden jedoch - mit Ausnahme der bereits erwähnten Polyurethanlösungen gemäß US 5,589,563 - nicht zur Beschichtung von medizinischen Geräten verwendet.

So beschreibt beispielsweise die DE 22 21 798 A ein Verfahren zur Herstellung stabiler und lichtbeständiger Lösungen von Polyurethanharnstoffen aus Prepolymeren mit endständigen Isocyanatgruppen und Diaminen in wenig polaren Lösungsmitteln, wobei Prepolymere aus
a) im wesentlichen linearen Polyhydroxylverbindungen mit Molekulargewichten von etwa 500 bis 5000,
b) gegebenenfalls niedermolekularen Dihydroxyverbindungen und
c) aliphatischen bzw. cycloaliphatischen Diisocyanaten, wobei das Molverhältnis von Hydroxyl- und Isocyanatgruppen zwischen ca. 1:1,5 und 1:5 liegt,
in einem Lösungsmittel(gemisch) aus gegebenenfalls chlorierten aromatischen und/oder chlorierten aliphatischen Kohlenwasserstoffen und primären, sekundären und/oder tertiären aliphatischen und/oder cycloaliphatischen Alkoholen mit Diaminen als Kettenverlängerer umgesetzt werden, wobei mindestens 80 Mol% des Kettenverlängerers 1,4-Diamino-cyclohexan mit einem cis/trans-Isomerenverhältnis zwischen 10/90 und 60/40 sind. Diese Polyurethanharnstoff-Lösungen werden zur Herstellung von lichtechten Folien und Überzügen verwendet.

Ferner beschreibt die DE 22 52 280 A ein Verfahren zur Beschichtung von textilen Unterlagen nach dem Umkehrverfahren mit Haft- und Deckstrichen aus Lösungen von aliphatischen, segmentierten Polyurethan-Elastomeren, die Polycarbonat-haltig sind.

Ferner beschreibt die EP 0 125 466 A ein Verfahren zur mehrstrichigen Umkehrbeschichtung von textilen, vorzugsweise bahnförmigen, Unterlagen zur Herstellung von Kunstleder aus zumindest einer Deckstrichlösung und mindestens einer Haftstrichlösung auf der Basis von Polyurethanen.

Aus der nicht vorveröffentlichten Europäischen Anmeldung Nr. 08153055.2 sind hydrophile Beschichtungen aus Polyurethanharnstoffen bekannt, welche auf eine speziellen Kombination von Polycarbonatpolyolen als Aufbaukomponenten und Copolymeren aus Ethylen- und Propylenoxid als Endgruppen basieren.

Es wurde nun gefunden, dass sich die mechanischen Eigenschaften dieser Beschichtungen verbessern lassen, wenn in der Polycarbonatpolyolkomponente gemäß der nicht vorveröffentlichten Europäischen Anmeldung Nr. 08153055.2 Polycarbonatpolyole eingesetzt werden, die Struktureinheiten der Formel (I) aufweisen

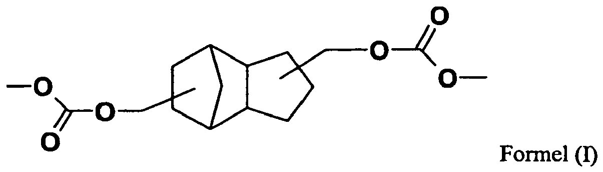

Gegenstand der vorliegenden Erfindung sind daher Polyurethanharnstofflösungen umfassend mindestens einen Polyurethanharnstoff, der Struktureinheiten der Formel (I) 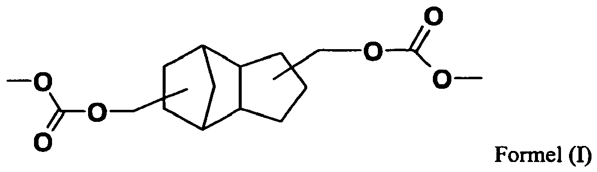 aufweist und mit wenigstens einer Copolymereinheit aus Polyethylenoxid und Polypropylenoxid terminiert ist.

Die durch die erfindungsgemäßen Lösungen erhältlichen Oberflächenbeschichtungen zeichnen sich durch eine hohe Hydrophilie, eine glatte Oberflächen und eine hohe Stabilität aus und können so beispielsweise die Bildung von Blutgerinnseln während der Behandlung mit dem medizinischen Gerät reduzieren.

Polyurethanharnstoffe im Sinne der vorliegenden Erfindung sind polymere Verbindungen, die
(a) mindestens zwei Urethangruppen enthaltende Wiederholungseinheiten der folgenden allgemeinen Struktur 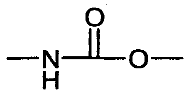 und
(b) mindestens eine Harnstoffgruppen enthaltende Wiederholungseinheit 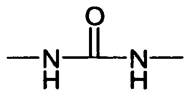 aufweisen.

Die erfindungsgemäßen Lösungen basieren auf Polyurethanharnstoffen der vorstehend genannten Art, welche im Wesentlichen keine ionische oder ionogene Modifizierung aufweisen. Hierunter wird im Rahmen der vorliegenden Erfindung verstanden, dass die erfindungsgemäß zu verwendenden Polyurethanharnstoffe im Wesentlichen keine ionischen Gruppen, wie insbesondere keine Sulfonat-, Carboxylat-, Phosphat- und Phosphonatgruppen, aufweisen.

Unter dem Begriff "im Wesentlichen keine ionische Gruppen" wird im Rahmen der vorliegenden Erfindung verstanden, dass die resultierenden Beschichtung des Polyurethanharnstoffs ionische Gruppen mit einem Anteil von im Allgemeinen höchstens 2,50 Gew.%, insbesondere höchstens 2,00 Gew.%, vorzugsweise höchstens 1,50 Gew.%, besonders bevorzugt höchstens 1,00 Gew.-%, speziell höchstens 0,50 Gew.%, noch spezieller keine ionische Gruppen aufweist. Damit ist insbesondere bevorzugt, dass der Polyurethanharnstoff keine ionischen Gruppen aufweist, da hohe Konzentration an Ionen in organischer Lösung dazu führen, dass das Polymer nicht mehr ausreichend löslich ist und somit keine stabilen Lösungen erhalten werden können. Falls das erfindungsgemäß verwendete Polyurethan ionische Gruppen aufweist, so handelt es sich bevorzugt um Carboxylate und Sulfonate.

Die erfindungswesentlichen Polyurethanharnstoffe der vorstehend genannten Art sind bevorzugt im Wesentlichen lineare Moleküle, können jedoch auch verzweigt sein, was jedoch weniger bevorzugt ist. Unter im Wesentlichen linearen Molekülen versteht man im Rahmen der vorliegenden Erfindung leicht anvernetzte Systeme, wobei die zugrundeliegende Polycarbonatpolyolkomponente eine mittlere Hydroxylfunktionalität von bevorzugt 1,7 bis 2,3, besonders bevorzugt 1,8 bis 2,2, ganz besonders bevorzugt 1,9 bis 2,1, aufweist.

Das zahlenmittlere Molekulargewicht der erfindungswesentlichen Polyurethanharstoffe beträgt vorzugsweise 1000 bis 200000 g/mol, besonders bevorzugt von 5000 bis 100000 g/mol. Dabei wird das zahlenmittlere Molekulargewicht gegen Polystyrol als Standard in Dimethylacetamid bei 30 °C gemessen.

Die erfindungsgemäßen Lösungen werden durch Umsetzung von Aufbaukomponenten hergestellt, die mindestens eine Polycarbonatpolyolkomponente a), mindestens eine Polyisocyanatkomponente b), mindestens eine Polyoxyalkylenetherkomponente c), mindestens eine Diamin- und/oder Aminoalkoholkomponente d) und gegebenenfalls eine weitere Polyolkomponente umfassen.

Daher ist ebenfalls ein Gegenstand der Erfindung ein Verfahren zur Herstellung der erfindungsgemäßen Lösungen, bei dem eine Polycarbonatpolyolkomponente a), mindestens eine Polyisocyanatkomponente b), mindestens eine Polyoxyalkylenetherkomponente c), mindestens eine Diamin- und/oder Aminoalkoholkomponente d) und gegebenenfalls eine weitere Polyolkomponente miteinander umgesetzt werden.

Komponente a) umfasst wenigstens ein Polycarbonatpolyol a1), welches durch Umsetzung von Kohlensäurederivaten, wie Diphenylcarbonat, Dimethylcarbonat oder Phosgen mit difunktionellen Alkoholen der Formel (II) 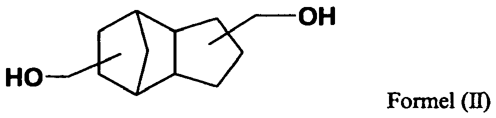 erhalten wird.

Zur Herstellung wird in einem Druckreaktor bei erhöhter Temperatur TCD Alkohol DM [3(4),8(9)-Bis(hydroxymethyl)-tricyclo(5.2.1.0/2.6)decan/ Tricyclodecandimethanol] mit Diphenylcarbonat, Dimethylcarbonat oder Phosgen umgesetzt. Bevorzugt ist die Umsetzung mit Dimethylcarbonat. Im Falle der Verwendung von Dimethylcarbonat wird das Spaltprodukt Methanol im Gemisch mit überzähligem Dimethylcarbonat per Destillation entfernt.

Diese Polycarbonatpolyole a1) auf Basis von Diolen der Formel (II) haben durch die OH-Zahl bestimmte Molekulargewichte von vorzugsweise 200 bis 10000 g/mol, besonders bevorzugt 300 bis 8000 g/mol und ganz besonders bevorzugt 400 bis 6000 g/mol.

Bevorzugt ist Komponente a) eine Mischung aus vorgenannten Polycarbonatpolyolen a1) auf Basis von Diolen der Formel (II) und weiteren Polycarbonatpolyolen a2).

Solche weiteren Polycarbonatpolyole a2) haben bevorzugt mittlere Hydroxylfunktionalitäten von 1,7 bis 2,3, besonders bevorzugt von 1,8 bis 2,2, ganz besonders bevorzugt von 1,9 bis 2,1.

Ferner weisen die Polycarbonatpolyole a2) durch die OH-Zahl bestimmte Molekulargewichte von vorzugsweise 400 bis 6000 g/mol, besonders bevorzugt 500 bis 5000 g/mol, insbesondere von 600 bis 3000 g/mol auf, die beispielsweise durch Reaktion von Kohlensäurederivaten, wie Diphenylcarbonat, Dimethylcarbonat oder Phosgen, mit Polyolen, vorzugsweise Diolen, erhältlich sind. Als derartige Diole kommen beispielsweise Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentan-1,3-diol, Di-, Tri- oder Tetraethylenglykol, Dipropylenglykol, Polypropylenglykole, Dibutylenglykol, Polybutylenglykole, Bisphenol A, Tetrabrombisphenol A aber auch Lacton-modifizierte Diole in Frage.

Diese Polycarbonatpolyole a2) enthalten bevorzugt 40 bis 100 Gew.% Hexandiol, bevorzugt 1,6-Hexandiol und/oder Hexandiol-Derivate, vorzugsweise solche, die neben endständigen OH-Gruppen Ether- oder Estergruppen aufweisen, z.B. Produkte, die durch Umsetzung von 1 Mol Hexandiol mit mindestens 1 Mol, bevorzugt 1 bis 2 Mol Caprolacton oder durch Veretherung von Hexandiol mit sich selbst zum Di- oder Trihexylenglykol erhalten wurden, als Aufbaukomponenten. Auch Polyether-Polycarbonatdiole können eingesetzt werden. Die Hydroxylpolycarbonate sollten im wesentlichen linear sein. Sie können jedoch gegebenenfalls durch den Einbau polyfunktioneller Komponenten, insbesondere niedermolekularer Polyole, leicht verzweigt werden. Hierzu eignen sich beispielsweise Glycerin, Hexantriol-1,2,6, Butantriol-1,2,4, Trimethylolpropan, Pentaerythrit, Chinit, Mannit, Sorbit, Methylglykosid oder 1,3,4,6-Dianhydrohexite. Bevorzugt sind solche Polycarbonate a2) auf Basis von Hexandiol-1,6, sowie modifizierend wirkenden Co-Diolen wie z. B. Butandiol-1,4 oder auch von ε-Caprolacton. Weitere bevorzugte Polycarbonatdiole a2) sind solche auf Basis von Mischungen aus Hexandiol-1,6 und Butandiol-1,4.

In einer bevorzugten Ausführungsform wird in a) eine Mischung eingesetzt aus den Polycarbonatpolyolen a1) und solchen Polycarbonatpolyolen a2) auf Basis von Hexandiol-1,6, Butandiol-1,4 oder deren Mischungen.

Im Fall von Mischungen der Bestandteile a1) und a2) beträgt der Anteil von a1) an der Mischung bevorzugt mindestens 5 mol%, besonders bevorzugt mindestens 10 mol%, bezogen auf die gesamte Molmenge an Polycarbonat.

Die erfindungswesentlichen Polyurethanharnstoffe weisen ferner Einheiten auf, welche auf mindestens ein Polyisocyanat als Aufbaukomponente b) zurückgehen.

Als Polyisocyanate b) können alle dem Fachmann bekannten aromatischen, araliphatischen, aliphatischen und cycloaliphatischen Isocyanate einer mittleren NCO-Funktionalität ≥ 1, bevorzugt ≥ 2 einzeln oder in beliebigen Mischungen untereinander eingesetzt werden, wobei es unerheblich ist, ob diese nach Phosgen- oder phosgen-freien Verfahren hergestellt wurden. Diese können auch Iminooxadiazindion-, Isocyanurat-, Uretdion-, Urethan-, Allophanat-, Biuret-, Harnstoff-, Oxadiazintrion, Oxazolidinon-, Acylharnstoff- und/oder Carbodiimid-Strukturen aufweisen. Die Polyisocyanate können einzeln oder in beliebigen Mischungen untereinander eingesetzt werden.

Bevorzugt werden Isocyanate aus der Reihe der aliphatischen oder cycloaliphatischen Vertreter eingesetzt, wobei diese ein Kohlenstoffgrundgerüst (ohne die enthaltenen NCO-Gruppen) von 3 bis 30, bevorzugt 4 bis 20 Kohlenstoffatomen aufweisen.

Besonders bevorzugte Verbindungen der Komponente b) entsprechen der vorstehend genannten Art mit aliphatisch und/oder cycloaliphatisch gebundene NCO-Gruppen wie beispielsweise Bis-(isocyanatoalkyl)ether, Bis- und Tris-(isocyanatoalkyl)benzole, -toluole, sowie -xylole, Propandiisocyanate, Butandiisocyanate, Pentandiisocyanate, Hexandiisocyanate (z.B. Hexamethylendiisocyanat, HDI), Heptandiisocyanate, Octandiisocyanate, Nonandiisocyanate (z.B. Trimethyl-HDI (TMDI) in der Regel als Gemisch der 2,4,4- und 2,2,4-Isomeren), Nonantriisocyanate (z.B. 4-Isocyanatomethyl-1,8-octandiisocyanat), Dekandiisocyanate, Dekantriisocyanate, Undekandiisocyanate, Undekantriisocyanate, Dodecandiisocyanate, Dodecantriisocyanate, 1,3- sowie 1,4-Bis-(isocyanatomethyl)cyclohexane (H₆XDI), 3-Isocyanatomethyl-3,5,5-trimethylcyclohexylisocyanat (Isophorondiisocyanat, IPDI), Bis-(4-isocyanatocyclohexyl)methan (H₁₂MDI) oder Bis-(isocyanatomethyl)norbornan (NBDI).

Ganz besonders bevorzugte Verbindungen der Komponente b) sind Hexamethylendiisocyanat (HDI), Trimethyl-HDI (TMDI), 2-Methylpentan-1,5-diisocyanat (MPDI), Isophorondiisocyanat (IPDI), 1,3- sowie 1,4-Bis(isocyanatomethyl)cyclohexan (H₆XDI), Bis(isocyanatomethyl)nor-bornan (NBDI), 3(4)-Isocyanatomethyl-1-methyl-cyclohexylisocyanat (IMCI) und/oder 4,4'-Bis-(isocyanatocyclohexyl)methan (H₁₂MDI) oder Gemische dieser Isocyanate. Weitere Beispiele sind Derivate aus den vorstehenden Diisocyanaten mit Uretdion-, Isocyanurat-, Urethan-, Allophanat-, Biuret-, Iminooxadiazindion- und/oder Oxadiazintrionstruktur mit mehr als zwei NCO-Gruppen.

Die Menge an Bestandteil b) bei der Herstellung der erfindungswesentlichen Polyurethanharnstoffe beträgt vorzugsweise 1,0 bis 3,5 mol, besonders bevorzugt 1,0 bis 3,3 mol, insbesondere 1,0 bis 3,0 mol, jeweils bezogen auf die Menge der Verbindungen der Komponente a).

Die erfindungswesentlichen Polyurethanharnstoffe weisen Einheiten auf, welche auf ein Copolymer aus Polyethylenoxid und Polypropylenoxid als Aufbaukomponente c) zurückgehen. Diese Copolymereinheiten liegen als Endgruppen in dem Polyurethanharnstoff vor und bewirken eine besonders vorteilhafte Hydrophilierung.

Solche nichtionisch hydrophilierenden Verbindungen c) sind beispielsweise einwertige, im statistischen Mittel 5 bis 70, bevorzugt 7 bis 55 Ethylenoxideinheiten pro Molekül aufweisende Polyalkylenoxidpolyetheralkohole, wie sie in an sich bekannter Weise durch Alkoxylierung geeigneter Startermoleküle zugänglich sind (z.B. in Ullmanns Enzyklopädie der technischen Chemie, 4. Auflage, Band 19, Verlag Chemie, Weinheim S. 31-38).

Geeignete Startermoleküle sind beispielsweise gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole. Besonders bevorzugt wird Diethylenglykolmonobutylether als Startermolekül verwendet.

Die Alkylenoxide Ethylenoxid und Propylenoxid können in beliebiger Reihenfolge oder auch im Gemisch bei der Alkoxylierungsreaktion eingesetzt werden können.

Bei den Polyalkylenoxidpolyetheralkoholen handelt es sich um gemischte Polyalkylenoxidpolyether aus Ethylenoxid und Propylenoxid, deren Alkylenoxideinheiten vorzugsweise zu mindestens 30 mol%, besonders bevorzugt zu mindestens 40 mol-% aus Ethylenoxideinheiten bestehen. Bevorzugte nichtionische Verbindungen sind monofunktionelle gemischte Polyalkylenoxidpolyether, die mindestens 40 mol% Ethylenoxid- und maximal 60 mol-% Propylenoxideinheiten bezogen auf den Gesamtanteil an Alkylenoxideinheiten aufweisen.

Das zahlenmittlere Molgewicht des Polyoxyalkylenethers beträgt vorzugsweise 500 g/mol bis 5000 g/mol, besonders bevorzugt 1000 g/mol bis 4000 g/mol, insbesondere 1000 bis 3000 g/mol.

Die Menge an Bestandteil c) bei der Herstellung der erfindungswesentlichen Polyurethanharnstoffe beträgt vorzugsweise 0,01 bis 0,5 mol, besonders bevorzugt 0,02 bis 0,4 mol, insbesondere 0,04 bis 0,3 mol, jeweils bezogen auf die Menge der Verbindungen der Komponente a).

Erfindungsgemäß konnte gezeigt werden, dass sich die Polyurethanharnstoffe mit Endgruppen, die auf gemischten Polyoxyalkylenether aus Polyethylenoxid und Polypropylenoxid basieren, insbesondere dazu eignen, Beschichtungen mit einer hohen Hydrophilie zu erzeugen.

Die erfindungswesentlichen Polyurethanharnstoffe weisen Einheiten auf, welche auf mindestens ein Diamin oder ein Aminoalkohol als Aufbaukomponente zurückgehen und als sogenannte Kettenverlängerer d) dienen.

Solche Kettenverlängerer sind beispielsweise Di- oder Polyamine sowie Hydrazide, z.B. Hydrazin, Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 1,3- und 1,4-Xylylendiamin, α,α,α',α'-Tetramethyl-1,3-und -1,4-xylylendiamin und 4,4'-Diaminodicyclohexylmethan, Dimethylethylendiamin, Hydrazin, A-dipinsäuredihydrazid, 1,4-Bis(aminomethyl)cyclohexan, 4,4'-Diamino-3,3'-dimethyldicyclohexyl-methan und andere (C₁ - C₄)-Di- und Tetraalkyldicyclo-hexylmethane, z. B. 4,4'-Diamino-3,5-diethyl-3',5'-diisopropyldicyclohexylmethan.

Als Diamine oder Aminoalkohole kommen im Allgemeinen niedermolekulare Diamine oder Aminoalkohole in Betracht, die aktiven Wasserstoff mit gegenüber NCO-Gruppen unterschiedlicher Reaktivität enthalten, wie Verbindungen, die neben einer primären Aminogruppe auch sekundäre Aminogruppen oder neben einer Aminogruppe (primär oder sekundär) auch OH-Gruppen aufweisen. Beispiele hierfür sind primäre und sekundäre Amine, wie 3-Amino-1-Methylaminopropan, 3-Amino-1-Ethylaminopropan, 3-Amino-1-cyclohexylaminopropan, 3-Amino-1-Methylaminobutan, weiterhin Aminoalkohole, wie N-Aminoethylethanolamin, Ethanolamin, 3-Aminopropanol, Neopentanolamin und besonders bevorzugt Diethanolamin.

Der Bestandteil d) der erfindungswesentlichen Polyurethanharnstoffe kann bei deren Herstellung als Kettenverlängerer eingesetzt werden.

Die Menge an Bestandteil d) bei der Herstellung der erfindungswesentlichen Polyurethanharnstoffe beträgt vorzugsweise 0,1 bis 1,5 mol, besonders bevorzugt 0,2 bis 1,3 mol, insbesondere 0,3 bis 1,2 mol, jeweils bezogen auf die Menge der Verbindungen der Komponente a).

In einer weiteren Ausführungsform umfassen die erfindungswesentlichen Polyurethanharnstoffe zusätzliche Einheiten, welche auf mindestens ein weiteres Polyol als Aufbaukomponente zurückgehen.

Die zum Aufbau der Polyurethanharnstoffe eingesetzten weiteren niedermolekularen Polyole e) bewirken in der Regel eine Versteifung und/oder eine Verzweigung der Polymerkette. Das Molekulargewicht beträgt vorzugsweise 62 bis 500 g/mol, besonders bevorzugt 62 bis 400 g/mol, insbesondere 62 bis 200 g/mol.

Geeignete Polyole können aliphatische, alicyclische oder aromatische Gruppen enthalten. Genannt seien hier beispielsweise die niedermolekularen Polyole mit bis zu etwa 20 Kohlenstoffatomen je Molekül, wie z. B. Ethyenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), sowie Trimethylolpropan, Glycerin oder Pentaerythrit und Mischungen dieser und gegebenenfalls auch weiterer niedermolekularer Polyole. Auch Esterdiole wie z.B. α-Hydroxybutyl-ε-hydroxycapronsäureester, ω-Hydroxyhexyl-γ-hydroxybuttersäure-ester, Adipinsäure-(β-hydroxyethyl)ester oder Terephthalsäure-bis(β-hydroxyethyl)-ester können verwendet werden.

Die Menge an Bestandteil e) bei der Herstellung der erfindungswesentlichen Polyurethanharnstoffe beträgt vorzugsweise 0,05 bis 1,0 mol, besonders bevorzugt 0,05 bis 0,5 mol, insbesondere 0,1 bis 0,5 mol, jeweils bezogen auf die Menge der Verbindungen der Komponente a).

Die Umsetzung der isocyanathaltigen Komponente b) mit den hydroxy- oder aminfunktionellen Verbindungen a), c), d) und gegebenenfalls e) erfolgt üblicherweise unter Einhaltung eines leichten NCO-Überschusses gegenüber den reaktiven Hydroxy- oder Aminverbindungen. Am Endpunkt der Reaktion durch Erreichen einer Zielviskosität verbleiben immer noch Reste an aktivem Isocyanat. Diese Reste müssen blockiert werden, damit nicht eine Reaktion mit großen Polymerketten stattfindet. Eine solche Reaktion führt zur dreidimensionalen Vernetzung und Vergelung des Ansatzes. Die Verarbeitung einer solchen Lösung ist nicht mehr möglich. Üblicherweise enthalten die Ansätze hohe Mengen an Alkoholen. Diese Alkohole blockieren innerhalb von mehreren Stunden Stehen oder Rühren des Ansatzes bei Raumtemperatur die noch verbliebenen Isocyanatgruppen.

Wurde bei der Herstellung der erfindungswesentlichen Polyurethanharnstoffe der Restisocyanatgehalt blockiert, weisen diese auch Monomere f) als Aufbaukomponenten auf, die sich jeweils an den Kettenenden befmden und diese abschließen.

Diese Aufbaukomponenten leiten sich zum einen von monofunktionellen, mit NCO-Gruppen reaktiven Verbindungen ab, wie Monoaminen, insbesondere mono-sekundären Aminen oder Monoalkoholen. Genannt seien hier beispielsweise Ethanol, n-Butanol, Ethylenglykol-monobutylether, 2-Ethylhexanol, 1-Octanol, 1-Dodecanol, 1-Hexadecanol, Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin und geeignete substituierte Derivate davon.

Da die Bausteine f) im Wesentlichen in den erfindungswesentlichen Polyurethanharnstoffen dazu verwendet werden, den NCO-Überschuss zu vernichten, hängt die erforderliche Menge im Wesentlichen von der Menge des NCO-Überschusses ab und kann nicht allgemein spezifiziert werden.

Bevorzugt wird während der Synthese auf diese Bausteine verzichtet. Noch nicht umgesetztes Isocyanat wird dabei vorzugsweise durch die in sehr großen Konzentrationen enthaltenen Lösungsmittelalkohole zu terminalen Urethanen umgesetzt.

Zur Herstellung der erfindungsgemäßen Polyurethanlösungen werden die Polycarbonatpolyolkomponente a), das Polyisocyanat, der monofunktionelle Polyetheralkohol und gegebenenfalls das Polyol in der Schmelze oder in Lösung miteinander umgesetzt, bis alle Hydroxylgruppen verbraucht sind.

Die dabei verwendete Stöchiometrie zwischen den einzelnen an der Umsetzung beteiligten Aufbaukomponenten ergibt sich aus den zuvor erwähnten Mengenverhältnissen.

Die Umsetzung erfolgt bei einer Temperatur von vorzugsweise 60 bis 110 °C, besonders bevorzugt 75 bis 110 °C, insbesondere 90 bis 110 °C, wobei Temperaturen um 110 °C aufgrund der Geschwindigkeit der Umsetzung bevorzugt ist. Höhere Temperaturen können ebenfalls angewendet werden, allerdings besteht dann im Einzelfall und in Abhängigkeit der einzelnen verwendeten Bestandteile das Risiko, dass Zersetzungsprozesse und Verfärbungen in dem entstehenden Polymer auftreten.

Bei dem Prepolymer aus Isocyanat und allen Hydroxylgruppen aufweisenden Komponenten ist die Umsetzung in Schmelze bevorzugt, allerdings besteht die Gefahr, dass es zu hohen Viskositäten der ausreagierten Gemische kommt. In diesen Fällen empfiehlt es sich auch, Lösemittel hinzuzugegeben. Es sollte aber möglichst nicht mehr als ungefähr 50 Gew.% Lösemittel enthalten sein, da andernfalls die Verdünnung die Reaktionsgeschwindigkeit deutlich verlangsamt.

Bei der Umsetzung von Isocyanat und den Hydroxylgruppen aufweisenden Komponenten kann die Reaktion in der Schmelze in einem Zeitraum von 1 Stunde bis 24 Stunden erfolgen. Geringe Zugaben von Lösungsmittelmengen führen zu einer Verlangsamung, wobei die Umsetzungszeiträume jedoch in den gleichen Zeiträumen liegen.

Die Reihenfolge der Zugabe bzw. Umsetzung der einzelnen Bestandteile kann von der zuvor angegebenen Reihenfolge abweichen. Dieses kann insbesondere dann von Vorteil sein, wenn die mechanischen Eigenschaften der resultierenden Beschichtungen verändert werden sollen. Wenn man beispielsweise alle Hydroxylgruppen aufweisenden Komponenten gleichzeitig umsetzt, entsteht ein Gemisch aus Hart- und Weichsegmenten. Wenn man beispielsweise das niedermolekulare Polyol nach der Polycarbonatpolyolkomponente zugibt, erhält man definierte Blöcke, was andere Eigenschaften der resultierenden Beschichtungen mit sich bringen kann. Die vorliegende Erfindung ist somit nicht auf eine beliebige Reihenfolge der Zugabe bzw. Umsetzung der einzelnen Bestandteile der Polyurethanbeschichtung beschränkt.

Dann wird weiteres Lösemittel zugesetzt und das gegebenenfalls gelöste Kettenverlängerungsdiamin bzw. der gelöste Kettenverlängerungsaminoalkohol (Aufbaukomponente (d)) zugegeben.

Die weitere Zugabe des Lösemittel erfolgt vorzugsweise schrittweise, um die Reaktion nicht unnötig zu verlangsamen, was bei einer vollständigen Zugabe der Lösemittelmenge beispielsweise am Anfang der Umsetzung passieren würde. Ferner ist man bei einem hohen Gehalt an Lösemittel zum Beginn der Reaktion an eine im Verhältnis niedrige Temperatur gebunden, welche von der Art des Lösemittels zumindest mitbestimmt wird. Auch dieses führt zu einer Verlangsamung der Reaktion.

Nach Erreichen der Zielviskosität können die noch verbleibenden Reste an NCO durch ein monofunktionelles aliphatisches Amin blockiert werden. Bevorzugt blockiert man die noch verbliebenen Isocyanatgruppen durch Umsetzung mit den im Lösungsmittelgemisch enthaltenen Alkoholen.

Als Lösungsmittel für die Herstellung und die Anwendung der erfindungsgemäßen Polyurethanhamstoff-Lösungen kommen alle denkbaren Lösungsmittel und Lösungsmittelgemische wie Dimethylformamid, N-Methylacetamid, Tetramethylharnstoff, N-Methylpyrrolidon, aromatische Lösungsmittel wie Toluol, lineare und cyclische Ester, Ether, Ketone und Alkohole in Frage. Beispiele für Ester und Ketone sind beispielsweise Ethylacetat, Butylacetat, Aceton, γ-Butyrolacton, Methylethylketon und Methylisobutylketon.

Bevorzugt sind Mischungen aus Alkoholen mit Toluol. Beispiele für die Alkohole, die gemeinsam mit dem Toluol verwendet werden, sind Ethanol, n-Propanol, iso-Propanol und 1-Methoxy-2-propanol.

Im Allgemeinen wird in der Umsetzung so viel Lösungsmittel eingesetzt, dass man ungefähr 10 bis 50 gew.%ige Lösungen, besonders bevorzugt ungefähr 15 bis 45 gew.%ige Lösungen, besonders bevorzugt ungefähr 20 bis 40 gew.-%ige Lösungen, erhält.

Der Feststoffgehalt der Polyurethanlösungen liegt im Allgemeinen im Bereich von 5 bis 60 Gew.-%, vorzugsweise 10 bis 40 Gew.%. Für Beschichtungsversuche können die Polyurethanlösungen beliebig mit Toluol/Alkohol-Gemischen verdünnt werden, um die Dicke der Beschichtung variabel einstellen zu können. Alle Konzentrationen von 1 bis 60 Gew.% sind möglich, bevorzugt sind Konzentrationen im Bereich 1 bis 40 Gew.-%.

Dabei können beliebige Schichtdicken erreicht werden wie beispielsweise einige 100 nm bis hinauf zu einigen 100 µm, wobei im Rahmen der vorliegenden Erfindung auch höhere und geringere Dicken möglich sind.

Die erfindungsgemäßen Polyurethanharnstofflösungen können ferner für den jeweils angestrebten Einsatzzweck übliche Bestandteile und Additive enthalten.

Ein Beispiel hierfür sind pharmakologische Wirkstoffe, Arzneimittel und Additive, welche die Freisetzung von pharmakologischen Wirkstoffen fördern ("drug-eluting-Additive").

Pharmakologische Wirkstoffe bzw. Arzneimittel, welche in den erfindungsgemäßen Beschichtungen auf den medizinischen Geräten verwendet werden können und damit in den erfindungsgemäßen Lösungen enthalten sein können, sind beispielsweise thromboresistente Mittel, antibiotische Mittel, Antitumormittel, Wachtumshormone, Antivirusmittel, antiangiogene Mittel, angiogene Mittel, antimitotische Mittel, entzündungshemmende Mittel, Zellzyklus-regulierende Mittel, genetische Mittel, Hormone, sowie deren Homologe, Derivate, Fragmente, pharmazeutische Salze und Kombinationen davon.

Spezifische Beispiele solcher pharmakologischen Wirkstoffe bzw. Arzneimittel schließen somit thromboresistente (nichtthrombogene) Mittel bzw. andere Mittel zur Unterdrückung einer akuten Thrombose, Stenose oder späten Re-Stenose der Arterien ein, beispielsweise Heparin, Streptokinase, Urokinase, Gewebe-Plasminogen-Aktivator, Anti-Thromboxan-B₂-Mittel; Anti-B-Thromoboglobulin, Prostaglandin-E, Aspirin, Dipyridimol, Anti-Thromboxan-A₂-Mittel, muriner monoklonaler Antikörper 7E3, Triazolopyrimidin, Ciprosten, Hirudin, Ticlopidin, Nicorandil usw. Ein Wachstumsfaktor kann als ebenfalls als ein Arzneimittel benutzt werden, um unterintimale fibromuskülare Hyperplasie an der arteriellen Stenosestelle zu unterdrücken, bzw. es kann jeder beliebige andere Hemmstoff des Zellwachstums an der Stenosestelle benutzt werden.

Der pharmakologische Wirkstoff bzw. das Arzneimittel kann auch aus einem Vasodilatator bestehen, um Vasospasmus entgegen zu wirken, zum Beispiel ein Antispasmusmittel wie Papaverin. Das Arzneimittel kann ein vasoaktives Mittel an sich sein, wie Calciumantagonisten, oder α- und β-adrenergische Agonisten oder Antagonisten. Zusätzlich kann das therapeutische Mittel ein biologisches Haftmittel wie Cyanoacrylat in medizinischer Qualität oder Fibrin, welche beispielsweise für das Ankleben einer Gewebeklappe an die Wand einer Koronararterie verwendet wird, sein.

Das therapeutische Mittel kann ferner ein antineoplastisches Mittel wie 5-Fluorouracil, vorzugsweise mit einem kontrollierenden freisetzenden Träger für das Mittel, sein (z.B. für die Anwendung eines fortdauernden kontrollierten freisetzenden antineoplastischen Mittels an einer Tumorstelle).

Das therapeutische Mittel kann ein Antibiotikum, vorzugsweise in Kombination mit einem kontrollierenden freisetzenden Träger für die fortdauernde Freisetzung aus der Beschichtung eines medizinischen Geräts an einen lokalisierten Infektionsherd innerhalb des Körpers, sein. Ähnlich kann das therapeutische Mittel Steroide für den Zweck des Unterdrückens einer Entzündung in lokalisiertem Gewebe oder aus anderen Gründen enthalten.

Spezifische Beispiele geeigneter Arzneimittel umfassen:
(a) Heparin, Heparinsulfat, Hirudin, Hyaluronsäure, Chondroitinsulfat, Dermatansulfat, Keratansulfat, lytische Mittel, einschließlich Urokinase und Streptokinase, deren Homologe, Analoge, Fragmente, Derivate und pharmazeutische Salze davon;
(b) antibiotischen Mitteln wie Penicilline, Cephalosporine, Vacomycine, Aminoglycoside, Quinolone, Polymyxine, Erythromycine; Tetracycline, Chloramphenicole, Clindamycine, Lincomycine, Sulfonamide, deren Homologe, Analoge, Derivate, pharmazeutische Salze und Mischungen davon;
(c) Paclitaxel, Docetaxel, Immunsuppressiva wie Sirolimus oder Everolimus, Alkylierungsmittel einschließlich Mechlorethamin, Chlorambucil, Cyclophosphamid, Melphalan und Ifosfamid; Antimetaboliten einschließlich Methotrexat, 6-Mercaptopurin, 5-Fluorouracil und Cytarabin; Pflanzenalkoide einschließlich Vinblastin; Vincristin und Etoposid; Antibiotika einschließlich Doxorubicin, Daunomycin, Bleomycin und Mitomycin; Nitrosurea einschließlich Carmustin und Lomustin; anorganische Ionen einschließlich Cisplatin; biologische Reaktionsmodifikatoren einschließlich Interferon; angiostatinische Mittel und endostatinische Mittel; Enzyme einschließlich Asparaginase; und Hormone einschließlich Tamoxifen und Flutamid, deren Homologe, Analoge, Fragmente, Derivate, pharmazeutische Salze und Mischungen davon;
(d) antivrale Mittel wie Amantadin, Rimantadin, Rabavirin, Idoxuridin, Vidarabin, Trifluridin, Acyclovir, Ganciclorir, Zidovudin, Phosphonoformate, Interferone, deren Homologe, Analoge, Fragmente, Derivate, pharmazeutische Salze und Mischungen davon; und
(e) entzündungshemmende Mittel wie beispielsweise Ibuprofen, Dexamethason oder Methylprednisolon.

Zur Erzeugung von Oberflächen mit bewuchshemmenden Eigenschaften können die erfindungsgemäßen Beschichtungszusammensetzungen die aus dem Stand der Technik bekannten bewuchshemmenden Wirkstoffe enthalten. Deren Anwesenheit verstärkt in der Regel die bereits hervorragenden bewuchshemmenden Eigenschaften der mit den findungsgemäßen Beschichtungszusammensetzungen selbst erzeugten Oberflächen.

Weitere Zusätze wie beispielsweise Antioxidantien oder Pigmente können ebenfalls verwendet werden. Darüber hinaus können gegebenenfalls noch weitere Zusätze wie Griffhilfsmittel, Farbstoffe, Mattierungsmittel, UV-Stabilisatoren, Lichtstabilisatoren, Hydrophobierungsmittel und/oder Verlaufshilfsmittel verwendet werden.

Die erfindungsgemäßen Polyurethanharnstofflösungen können dazu verwendet werden, eine Beschichtung zum Beispiel auf einem medizinischen Gerät zu bilden.

Die Bezeichnung "medizinisches Gerät" ist im Rahmen der vorliegenden Erfindung breit zu verstehen. Geeignete, nicht einschränkende Beispiele für medizinische Geräte (einschließlich Instrumente) sind Kontaktlinsen; Kanülen; Kathetern, zum Beispiel urologische Kathetern wie Blasenkathetern oder Harnleiterkathetern; zentralvenöse Katheter; venöse Kathetern oder Einlass- bzw. Auslass-Kathetem; Dilationsballons; Kathetern für die Angioplastie und die Biopsie; Kathetern, die für das Einbringen eines Stents, eines Propf- oder eines Kavafilters verwendet werden; Ballonkathetern oder andere dehnbare medizinische Geräte; Endoskope; Larnygoskope; Trachealgeräte wie Endotrachealschläuche, Atemgeräte und andere Trachealabsauggeräte; bronchoalveolare Spülungskathetem; Kathetern, die bei der Koronarangioplastie verwendet werden; Führungsstäbe, Einführer und Ähnliches; Gefäßpfropfen; Schrittmacherteile; Cochleaimplantate; Zahnimplantatschläuche für die Nahrungszufuhr, Dränageschläuche; und Führungsdrähte.

Darüber hinaus können die erfindungsgemäßen Lösungen zur Herstellung von Schutzbeschichtungen, zum Beispiel für Handschuhe, Stents und andere Implantate; extrakorporale (außerkörperliche) Blutschläuche (Blutführungsrohre); Membrane, zum Beispiel für die Dialyse; Blutfilter; Geräte für die Kreislaufunterstützung; Verbandsmaterial für die Wundpflege; Harnbeutel und Stomabeutel verwendet werden. Eingeschlossen sind auch Implantate, die ein medizinisch wirksames Mittel enthalten, wie medizinisch wirksame Mitteln für Stents oder für Ballonoberflächen oder für Kontrazeptiva.

Üblicherweise wird das medizinisches Gerät aus Kathetern, Endoskopen, Laryngoskopen, Endotrachealschläuchen, Ernährungsschläuchen, Führungsstäben, Stents, und andere Implantate gebildet.

Zusätzlich zu den hydrophilen Eigenschaften der Verbesserung der Gleitfähigkeit zeichnen sich die erfindungsgemäß vorgesehenen Beschichtungszusammensetzungen auch durch eine hohe Blutverträglichkeit aus. Hierdurch ist auch ein Arbeiten mit diesen Beschichtungen besonders im Blutkontakt vorteilhaft. Die Materialien zeigen im Vergleich zu Polymeren des Standes der Technik eine reduzierte Gerinnungsneigung im Blutkontakt.

Wirkstofffreisetzende Systeme basierend auf den erfindungsgemäßen hydrophilen Beschichtungsmaterialien sind auch außerhalb der Medizintechnik denkbar, beispielsweise für Anwendungen im Pflanzenschutz als Trägermaterial für Wirkstoffe. Die gesamte Beschichtung kann dann als wirkstofffreisetzendes System betrachtet werden und beispielsweise zur Beschichtung von Saatgut (Samenkörner) eingesetzt werden. Durch die hydrophilen Eigenschaften der Beschichtung kann der enthaltene Wirkstoff im feuchten Erdreich austreten und seine bestimmungsgemäße Wirkung entfalten ohne dass die Keimfähigkeit des Saatgutes beeinträchtigt wird. Im trockenen Zustand bindet das Beschichtungsmittel den Wirkstoff jedoch sicher an das Saatgut, so dass beispielsweise beim Einschießen des Samenkorns mit der Ausbringmaschine in den Boden der Wirkstoff nicht abgelöst wird, wodurch er unerwünschte Wirkungen z.B. auf die vorhandene Fauna entfalten könnte (Bienengefährdung durch Insektizide, die an sich den Insektenbefall des Samenkorns im Boden verhindern sollen).

Über die Anwendung als Beschichtung für medizinische Geräte hinaus können die erfindungsgemäßen Polyurethanlösungen auch für weitere technische Anwendungen im nicht-medizinischen Bereich benutzt werden.

So dienen die erfindungsgemäße Polyurethanlösungen zu Herstellung von Beschichtungen als Schutz von Oberflächen vor Beschlagen mit Feuchtigkeit, zur Herstellung von leicht zu reinigenden oder von selbstreinigenden Oberflächen. Diese hydrophilen Beschichtungen verringern auch die Aufnahme von Schmutz und verhindern die Bildung von Wasserflecken. Denkbare Anwendungen im Außenbereich sind beispielsweise Fensterscheiben und Dachluken, Glasfassaden oder Plexiglasdächer. Im Innenbereich können solche Materialien für die Beschichtung von Oberflächen im Sanitärbereich benutzt werden. Weitere Anwendungen sind die Beschichtung von Brillengläsern oder von Verpackungsmaterialien wie Lebensmittelverpackungen zur Vermeidung von Feuchtigkeitsbeschlag oder Tropfenbildung durch kondensiertes Wasser.

Die erfindungsgemäße Polyurethanlösungen eignen sich ebenso zur Ausrüstung von Oberflächen im Kontakt mit Wasser zur Verminderung des Bewuchses. Diesen Effekt nennt man auch Antifoulingeffekt. Eine sehr wichtige Anwendung dieses Antifoulingeffektes ist im Bereich der Unterwasseranstriche von Schiffsrümpfen. Schiffsrümpfe ohne Antifoulingausrüstung werden sehr schnell von Meeresorganismen bewachsen, was durch erhöhte Reibung zu einer Verringerung der möglichen Geschwindigkeit und einem höheren Treibstoffverbrauch führt. Die erfindungsgemäßen Beschichtungsmaterialien reduzieren oder verhindern den Bewuchs mit Meeresorganismen und verhindern die oben beschriebenen Nachteile dieses Bewuchses. Weitere Anwendungen im Bereich der Antifoulingbeschichtungen sind Artikel für die Fischerei wie Fischernetze sowie alle metallischen Substrate im Unterwassereinsatz wie Rohrleitungen, Bohrinseln, Schleusenkammern und - tore etc. Schiffsrümpfe, die mit den erfindungsgemäßen Beschichtungsmaterialien erzeugte Oberflächen insbesondere unterhalb der Wasserlinie aufweisen, besitzen auch einen reduzierten Reibungswiderstand, so dass derartig ausgerüstete Schiffe entweder einen reduzierten Brennstoff-Verbrauch aufweisen oder höhere Geschwindigkeiten erreichen. Dies ist insbesondere im Sportbootbereich und Yachtbau von Interesse.

Ein weiteres wichtiges Anwendungsgebiet der obengenannten hydrophilen Beschichtungsmaterialien ist die Druckindustrie. Hydrophobe Oberflächen können durch die erfindungsgemäßen Beschichtungen hydrophilisiert werden und sind dadurch mit polaren Druckfarben bedruckbar bzw. können mittels Tintenstrahltechnik aufgebracht werden.

Ein weiterer Anwendungsbereich der erfindungsgemäßen hydrophilen Beschichtungen sind Formulierungen für kosmetischen Anwendungen.

Beschichtungen aus den erfmdungsgemäße Polyurethanlösungen können mittels verschiedener Verfahren aufgebracht werden. Geeignete Beschichtungstechniken sind hierfür beispielsweise Rakeln, Drucken, Transferbeschichten, Sprühen, Spincoating oder Tauchen.

Beschichtet werden können vielerlei Substrate wie Metalle, Textilien, Keramiken und Kunststoffe. Bevorzugt ist die Beschichtung von medizinischen Geräten, die aus Kunststoff oder Metallen gefertigt sind. Als Metalle können beispielsweise genannt werden: Medizinischer Edelstahl und Nickel-Titan-Legierungen. Es sind viele Polymermaterialien denkbar, aus denen das medizinische Geräte aufgebaut sein kann, beispielsweise Polyamid; Polystyrol; Polycarbonat; Polyether; Polyester; Polyvinylacetat; natürliche und synthetische Kautschuke; Blockcopolymere aus Styrol und ungesättigten Verbindungen wie Ethylen, Butylen und Isopren; Polyethylen oder Copolymeren aus Polyethylen und Polypropylen; Silikon; Polyvinylchlorid (PVC) und Polyurethanen. Zur besseren Haftung des hydrophilen Polyurethane auf dem medizinischen Gerät können als Untergrund vor dem Auftragen dieser hydrophilen Beschichtungsmaterialien noch weitere geeignete Beschichtungen aufgetragen werden.

### Beispiele

Die Ermittlung des NCO-Gehaltes der in den Beispielen und Vergleichsbeispielen beschriebenen Harze erfolgte durch Titration gemäß DIN EN ISO 11909.

Die Bestimmung der Festkörpergehalte erfolgte nach DIN-EN ISO 3251. Es wurden 1 g Polyurethandispersion bei 115 °C bis zur Gewichtskonstanz (15-20 min) mittels eines Infrarottrockners getrocknet.

Die Messung der mittleren Teilchengrößen der Polyurethandispersionen erfolgt mit Hilfe des High Performance Particle Sizer (HPPS 3.3) der Firma Malvern Instruments.

Die in % angegebenen Mengenangaben verstehen sich, wenn nicht anders vermerkt, als Gew.-% und beziehen sich auf die erhaltene wässrige Dispersion.

Die Zugfestigkeiten wurden nach DIN 53504 bestimmt.

Viskositätsmessungen wurden mit dem Physics MCR 51 Rheometer der Firma Anton Paar GmbH, Ostfildern, Deutschland, durchgeführt.

### Verwendete Substanzen und Abkürzungen:

| | |
|---|---|
| Desmophen C2200: | Polycarbonatpolyol, OH-Zahl 56 mg KOH/g, zahlenmittleres Molekulargewicht 2000 g/mol (Bayer MaterialScience AG, Leverkusen, DE) |
| | |
| Desmophen C1200: | Polycarbonatpolyol, OH-Zahl 56 mg KOH/g, zahlenmittleres Molekulargewicht 2000 g/mol (Bayer MaterialScience AG, Leverkusen, DE) |
| | |
| Desmophen XP 2613 | Polycarbonatpolyol, OH-Zahl 56 mg KOH/g, zahlenmittleres Molekulargewicht 2000 g/mol (Bayer MaterialScience AG, Leverkusen, DE) |
| | |
| Polyether LB 25: | (monofunktioneller Polyether auf Ethylenoxid/Propylenoxidbasis zahlenmittleres Molekulargewicht 2250 g/mol, OH-Zahl 25 mg KOH/g (Bayer MaterialScience AG, Leverkusen, DE) |
| | |
| TCD-Alkohol DM | 3(4),8(9)-Bis(hydroxymethyl)tricyclo(5.2.1.0/2.6)decan/ Tricyclodecandimethanol der Firma Celanese Corp., Dallas, USA |

### Beispiel 1:

### Herstellung eines cycloaliphatischen Polycarbonatdiols auf Basis TCD-Alkohol DM mit einem zahlenmittleren Molekulargewicht von 1.300 g/mol

In einem 16 1 Druckreaktor mit Destillationsaufsatz, Rührer und Vorlage wurden 5.436 g TCD Alkohol DM mit 1,2 g Yttrium(III)acetylacetonat sowie 3.810 g Dimethylcarbonat bei 80 °C vorgelegt. Anschließend wurde unter Stickstoffatmosphäre das Reaktionsgemisch in 2 h auf 135 °C aufgeheizt und dort unter Rühren 24 h gehalten wobei der Druck auf 6,3 bar (absolut) anstieg. Danach wurde auf 60 °C abgekühlt und belüftet. Dann wurde das Spaltprodukt Methanol im Gemisch mit Dimethylcarbonat per Destillation entfernt, wobei die Temperatur schrittweise auf 150 °C erhöht wurde. Es wurde dann noch 4 Stunden bei 150 °C gerührt, anschließend auf 180 °C aufgeheizt und dann noch 4 h bei 180 °C gerührt. Dann wurde die Temperatur auf 90 °C reduziert und ein Stickstoffstrom (5 1/h) durch das Reaktionsgemisch hindurchgeleitet, während der Druck auf 20 mbar abgesenkt wurde. Danach wurde die Temperatur binnen 4 h auf 180 °C erhöht und dort 6 h gehalten. Dabei erfolgte die weitere Entfernung von Methanol im Gemisch mit Dimethylcarbonat aus dem Reaktionsgemisch.

Nach Belüftung und Abkühlung des Reaktionsansatzes auf Raumtemperatur, wurde ein gelbliches, festes Polycarbonatdiol mit folgenden Kennzahlen erhalten:
Mₙ = 1.290 g/mol; OH-Zahl = 87 mg KOH/g;

### Beispiel 2:

### Herstellung eines cycloaliphatischen Polycarbonatdiols auf Basis TCD-Alkohol DM mit einem zahlenmittleren Molekulargewicht von 1.000 g/mol

Vorgehen wie in Beispiel 1, wobei 5.436 g TCD-Alkohol DM, 1,2 g Yttrium(III)acetylacetonat und 2.931 g Dimethylcarbonat verwendet wurden.

Man erhielt ein gelbliches, hochviskoses Polycarbonatdiol mit folgenden Kennzahlen: Mₙ = 1000 g/mol; OH-Zahl = 112 mg KOH/g

### Beispiel 3:

### Herstellung eines cycloaliphatischen Polycarbonatdiols auf Basis TCD-Alkohol DM mit einem zahlenmittleren Molekulargewicht von ca. 500 g/mol

Vorgehen wie in Beispiel 1, wobei 7790 g TCD-Alkohol DM, 1,68 g Yttrium(III)acetylacetonat und 3096 g Dimethylcarbonat verwendet wurden.

Man erhielt ein gelbliches, hochviskoses Polycarbonatdiol mit folgenden Kennzahlen: Mₙ = 496 g/mol; OH-Zahl = 226 mg KOH/g; Vislosität bei 75 °C = 138.400 mPas.

### Beispiel 4:

### Herstellung eines (cyclo)aliphatischen Polycarbonatdiols auf Basis TCD-Alkohol DM und 1,4-Butandiol mit einem zahlenmittleren Molekulargewicht von ca. 1.000 g/mol

Vorgehen wie in Beispiel 1, wobei 5951 g TCD-Alkohol DM, 2732 g 1,4-Butandiol, 2,0 g Yttrium(III)acetylacetonat und 6842 g Dimethylcarbonat verwendet wurden.

Man erhielt ein farbloseses, Polycarbonatdiol mit folgenden Kennzahlen: Mₙ = 943 g/mol; OH-Zahl = 119 mg KOH/g; Viskosität bei 75 °C = 15.130 mPas.

### Beispiel 5: (Vergleich)

195,4 g Desmophen C 2200, 30,0 g LB 25 und 47,8 g 4,4'-Bis(isocyanatocyclohexyl)methan (H₁₂MDI) wurden bei 110 °C bis zu einem konstanten NCO-Gehalt von 2,4 % umgesetzt. Man ließ abkühlen und verdünnte mit 350,0 g Toluol und 200 g iso-Propanol. Bei Raumtemperatur wurden eine Lösung von 11,8 g Isophorondiamin in 94,0 g 1-Methoxypropanol-2 zugegeben. Nach beendetem Aufbau des Molgewichtes und Erreichen des gewünschten Viskositätsbereiches rührte man weitere 5 Stunden, um den restlichen Isocyanatgehalt mit iso-Propanol zu blockieren. Man erhielt 929 g einer 31,9%igen Polyurethanharnstofflösung in Toluol/iso-Propanol/1-Methoxypropanol-2 mit einer Viskosität von 37100 mPas bei 22 °C.

### Beispiel 6: (erfindungsgemäß)

175,8 g Desmophen C 2200, 12,7 g Polycarbonatdiol des Beispiels 1, 30,0 g LB 25 und 47,8 g 4,4'-Bis(isocyanatocyclohexyl)methan (H₁₂MDI) wurden bei 110 °C bis zu einem konstanten NCO-Gehalt von 2,5 % umgesetzt. Man ließ abkühlen und verdünnte mit 340,0 g Toluol und 200 g iso-Propanol. Bei Raumtemperatur wurden eine Lösung von 12,4 g Isophorondiamin in 93,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 912 g einer 30,8%igen Polyurethanharnstofflösung in Toluol/iso-Propanol/1-Methoxypropanol-2 mit einer Viskosität von 28500 mPas bei 22 °C.

### Beispiel 7: (erfindungsgemäß)

146,6 g Desmophen C 2200, 31,7 g Polycarbonatdiol des Beispiels 1, 30,0 g LB 25 und 47,8 g 4,4'-Bis(isocyanatocyclohexyl)methan (H₁₂MDI) wurden bei 110 °C bis zu einem konstanten NCO-Gehalt von 2,5 % umgesetzt. Man ließ abkühlen und verdünnte mit 330,0 g Toluol und 200 g iso-Propanol. Bei Raumtemperatur wurden eine Lösung von 12,5 g Isophorondiamin in 98,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 897 g einer 30,4%igen Polyurethanharnstofflösung in Toluol/iso-Propanol/1-Methoxypropanol-2 mit einer Viskosität von 20600 mPas bei 22 °C.

### Beispiel 8: (erfindungsgemäß)

97,8 g Desmophen C 2200, 63,6 g Polycarbonatdiol des Beispiels 1, 30,0 g LB 25 und 47,8 g 4,4'-Bis(isocyanatocyclohexyl)methan (H₁₂MDI) wurden bei 110 °C bis zu einem konstanten NCO-Gehalt von 2,7 % umgesetzt. Man ließ abkühlen und verdünnte mit 335,0 g Toluol und 185 g iso-Propanol. Bei Raumtemperatur wurden eine Lösung von 12,7 g Isophorondiamin in 99,0 g 1-Methoxypropanol-2 zugegeben. Nach beendetem Aufbau des Molgewichtes und Erreichen des gewünschten Viskositätsbereiches rührte man weitere 2 Stunden, um den restlichen Isocyanatgehalt mit iso-Propanol zu blockieren. Man erhielt 871 g einer 29,3%igen Polyurethanharnstofflösung in Toluol/iso-Propanol/1-Methoxypropanol-2 mit einer Viskosität von 17000 mPas bei 22 °C.

### Beispiel 9: (erfindungsgemäß)

175,8 g Desmophen C 2200, 9,8 g Polycarbonatdiol des Beispiels 4, 30,0 g LB 25 und 47,8 g 4,4'-Bis(isocyanatocyclohexyl)methan (H₁₂MDI) wurden bei 110 °C bis zu einem konstanten NCO-Gehalt von 2,5 % umgesetzt. Man ließ abkühlen und verdünnte mit 350,0 g Toluol und 200 g iso-Propanol. Bei Raumtemperatur wurden eine Lösung von 12,3 g Isophorondiamin in 98,0 g 1-Methoxypropanol-2 zugegeben. Nach beendetem Aufbau des Molgewichtes und Erreichen des gewünschten Viskositätsbereiches rührte man weitere 5 Stunden, um den restlichen Isocyanatgehalt mit iso-Propanol zu blockieren. Man erhielt 924 g einer 30,1%igen Polyurethanharnstofflösung in Toluol/iso-Propanol/1-Methoxypropanol-2 mit einer Viskosität von 18900 mPas bei 22 °C.

### Beispiel 10: (erfindungsgemäß)

146,6 g Desmophen C 2200, 24,4 g Polycarbonatdiol des Beispiels 4, 30,0 g LB 25 und 47,8 g 4,4'-Bis(isocyanatocyclohexyl)methan (H₁₂MDI) wurden bei 110 °C bis zu einem konstanten NCO-Gehalt von 2,6 % umgesetzt. Man ließ abkühlen und verdünnte mit 335,0 g Toluol und 190 g iso-Propanol. Bei Raumtemperatur wurden eine Lösung von 12,5 g Isophorondiamin in 100,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 886 g einer 30,1 %igen Polyurethanhamstofflösung in Toluol/iso-Propanol/1-Methoxypropanol-2 mit einer Viskosität von 20600 mPas bei 22 °C.

### Beispiel 11: (erfmdungsgemäß)

97,8 g Desmophen C 2200, 48,9 g Polycarbonatdiol des Beispiels 4, 30,0 g LB 25 und 47,8 g 4,4'-Bis(isocyanatocyclohexyl)methan (H₁₂MDI) wurden bei 110°C bis zu einem konstanten NCO-Gehalt von 2,8 % umgesetzt. Man ließ abkühlen und verdünnte mit 325,0 g Toluol und 175 g iso-Propanol. Bei Raumtemperatur wurden eine Lösung von 12,7 g Isophorondiamin in 98,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 835 g einer 29,0%igen Polyurethanharnstofflösung in Toluol/iso-Propanol/1-Methoxypropanol-2 mit einer Viskosität von 27400 mPas bei 22 °C.

### Beispiel 12: Kontaktwinkel und 100%-Module von Vergleichsbeispiel 4 gegen erfindungsgemäße Beispiele 5-10

### 1. Herstellung der Beschichtungen für die Messung des statischen Kontaktwinkels

Die Beschichtungen zur Messung des statischen Kontaktwinkels wurden auf Glasobjektträger der Größe 25x75 mm mit Hilfe eines Spincoaters (RC5 Gyrset 5, Karl Süss, Garching, Deutschland) hergestellt. Ein Objektträger wurde hierzu auf dem Probenteller des Spincoaters eingespannt und mit ca. 2,5 - 3 g organischer 15%iger Polyurethanlösung homogen bedeckt Alle organischen Polyurethanlösungen wurden mit einem Lösungsmittelgemisch aus 65 Gew% Toluol und 35 Gew% iso-Propanol auf einen Polymergehalt von 1 Gew% verdünnt. Durch Rotation des Probentellers für 20 sec bei 1300 Umdrehungen pro Minute erhielt man eine homogene Beschichtung, die 1 h bei 100 °C und danach 24 h bei 50 °C getrocknet wurde. Die erhaltenen beschichteten Objektträger wurden direkt einer Kontaktwinkelmessung unterzogen.

Von den erhaltenen Beschichtungen auf den Objektträgern wurde eine statische Kontaktwinkelmessung durchgeführt. Mittels des Video- Randwinkelmessgerätes OCA20 der Firma Dataphysics mit rechnergesteuerten Spritzen wurde auf das Muster 10 Tropfen Milliporewasser aufgesetzt und deren statischer Benetzungsrandwinkel gemessen. Zuvor wurde mittels eines Antistatikföns die statische Aufladung (falls vorhanden) auf der Probenoberfläche entfernt.

### 2. Herstellung der Beschichtungen für die Messung des 100%-Moduls

Auf Trennpapier werden mit einem 200µm-Rakel Schichten hergestellt, die 15 min bei 100 °C getrocknet werden. Danach erfolgt Trocknung für 15 min bei 100 °C. Ausgestanzte Formkörper werden nach DIN 53504 untersucht.

### 3. Untersuchungsergebnisse

**Tabelle 1: Kontaktwinkel und 100%-Module der Filme aus Materialien der Beispiele 5-11**

| Beispiel-Nr. | Kontaktwinkel (°) | 100%-Modul (N/mm²) |
|---|---|---|
| Vergleichsbeispiel 5 | 21 | 2,3 |
| Beispiel 6 | 33 | 2,5 |
| Beispiel 7 | 33 | 2,6 |
| Beispiel 8 | 24 | 6,4 |
| Beispiel 9 | 30 | 2,5 |
| Beispiel 10 | 30 | 2,9 |
| Beispiel 11 | 41 | 4,3 |

Die erfindungsgemäßen Beispiele 6 bis 11 unterscheiden sich dadurch, dass im Vergleich zum Vergleichsbeispiel 5 ein Teil des Polycarbonatdiols Desmophen C2200 durch das erfindungswesentliche Polycarbonatdiol ersetzt wurde. Die Materialien zeigen als Beschichtung ähnlich hydrophile Eigenschaften wie das Vergleichsbeispiel 5. Die 100%-Module sind alle höher als dasjenige des Vergleichsbeispiels 5.

### Beispiel 13: (Vergleich)

195,4 g Desmophen C 1200, 30,0 g LB 25 und 47,8 g 4,4'-Bis(isocyanatocyclohexyl)methan (H₁₂MDI) wurden bei 110 °C bis zu einem konstanten NCO-Gehalt von 2,4 % umgesetzt. Man ließ abkühlen und verdünnte mit 350,0 g Toluol und 200 g iso-Propanol. Bei Raumtemperatur wurden eine Lösung von 13,3 g Isophorondiamin in 100 g 1-Methoxypropanol-2 zugegeben. Nach beendetem Aufbau des Molgewichtes und Erreichen des gewünschten Viskositätsbereiches rührte man weitere 4,5 Stunden, um den restlichen Isocyanatgehalt mit iso-Propanol zu blockieren. Man erhielt 936 g einer 30,6%igen Polyurethanharnstofflösung in Toluol/iso-Propanol/1-Methoxypropanol-2 mit einer Viskosität von 10200 mPas bei 22 °C.

### Beispiel 14: (erfindungsgemäß)

97,8 g Desmophen C 1200, 24,5 g Polycarbonatdiol des Beispiels 3, 30,0 g LB 25 und 47,8 g 4,4'-Bis(isocyanatocyclohexyl)methan (H₁₂MDI) wurden bei 110°C bis zu einem konstanten NCO-Gehalt von 3,3 % umgesetzt. Man ließ abkühlen und verdünnte mit 250,0 g Toluol und 150 g iso-Propanol. Bei Raumtemperatur wurden eine Lösung von 13,1 g Isophorondiamin in 100 g 1-Methoxypropanol-2 zugegeben. Nach beendetem Aufbau des Molgewichtes und Erreichen des gewünschten Viskositätsbereiches rührte man weitere 3 Stunden, um den restlichen Isocyanatgehalt mit iso-Propanol zu blockieren. Man erhielt 713 g einer 30,4%igen Polyurethanharnstofflösung in Toluol/iso-Propanol/1-Methoxypropanol-2 mit einer Viskosität von 29200 mPas bei 22 °C.

### Beispiel 15: (erfindungsgemäß)

130,2 g Desmophen C 1200, 16,3 g Polycarbonatdiol des Beispiels 3, 30,0 g LB 25 und 47,8 g 4,4'-Bis(isocyanatocyclohexyl)methan (H₁₂MDI) wurden bei 110 °C bis zu einem konstanten NCO-Gehalt von 2,9 % umgesetzt. Man ließ abkühlen und verdünnte mit 320,0 g Toluol und 170 g iso-Propanol. Bei Raumtemperatur wurden eine Lösung von 13,2 g Isophorondiamin in 99 g 1-Methoxypropanol-2 zugegeben. Nach beendetem Aufbau des Molgewichtes und Erreichen des gewünschten Viskositätsbereiches rührte man weitere 3 Stunden, um den restlichen Isocyanatgehalt mit iso-Propanol zu blockieren. Man erhielt 827 g einer 29,0%igen Polyurethanharnstofflösung in Toluol/iso-Propanol/1-Methoxypropanol-2 mit einer Viskosität von 31900 mPas bei 22 °C.

### Beispiel 16: Kontaktwinkel und 100%-Module von Vergleichsbeispiel 13 gegen erfinderische Beispiele 14 und 15

Die Herstellung der Beschichtungen sowie die Ermittlung der Kontaktwinkel und 100%-Module erfolgt wie im Beispiel 12 beschrieben.

**Tabelle 2: Kontaktwinkel und 100%-Module der Filme aus Materialien der Beispiele 13, 14 und 15**

| Beispiel-Nr. | Kontaktwinkel (°) | 100%-Modul (N/mm²) |
|---|---|---|
| Vergleichsbeispiel 13 | 11 | 1,8 |
| Beispiel 14 | 18 | 6,1 |
| Beispiel 15 | 18 | 5,0 |

Die erfindungsgemäßen Beispiele 14 und 15 enthalten im Vergleich zum Vergleichsbeispiel 13 Anteile des erfindungswesentlichen Polycarbonatdiols. Die Oberfläche der Beschichtung ist nach wie vor sehr hydrophil, während sich das 100%-Modul in etwa verdreifacht.

### Beispiel 17: (Vergleich).

195,4 g Desmophen C 2200, 40,0 g LB 25 und 47,8 g 4,4'-Bis(isocyanatocyclohexyl)methan (H₁₂MDI) wurden bei 110 °C bis zu einem konstanten NCO-Gehalt von 2,2 % umgesetzt. Man ließ abkühlen und verdünnte mit 350,0 g Toluol und 200 g iso-Propanol. Bei Raumtemperatur wurden eine Lösung von 12,0 g Isophorondiamin in 100,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 945 g einer 31,6%igen Polyurethanhamstofflösung in Toluol/iso-Propanol/1-Methoxypropanol-2 mit einer Viskosität von 19300 mPas bei 22 °C.

### Beispiel 18: (erfindungsgemäß)

97,8 g Desmophen C 2200, 48,9 g Polycarbonatdiol des Beispiels 4, 40,0 g LB 25 und 47,8 g 4,4'-Bis(isocyanatocyclohexyl)methan (H₁₂MDI) wurden bei 110 °C bis zu einem konstanten NCO-Gehalt von 2,7 % umgesetzt. Man ließ abkühlen und verdünnte mit 320,0 g Toluol und 180 g iso-Propanol. Bei Raumtemperatur wurden eine Lösung von 12,3 g Isophorondiamin in 100 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 847 g einer 29,6%igen Polyurethanharnstofflösung in Toluol/iso-Propanol/1-Methoxypropanol-2 mit einer Viskosität von 9600 mPas bei 22 °C.

### Beispiel 19: (erfindungsgemäß)

97,8 g Desmophen C 2200, 24,5 g Polycarbonatdiol des Beispiels 3, 40,0 g LB 25 und 47,8 g 4,4'-Bis(isocyanatocyclohexyl)methan (H₁₂MDI) wurden bei 110 °C bis zu einem konstanten NCO-Gehalt von 3,0 % umgesetzt. Man ließ abkühlen und verdünnte mit 300,0 g Toluol und 180 g iso-Propanol. Bei Raumtemperatur wurden eine Lösung von 12,5 g Isophorondiamin in 100 g 1-Methoxypropanol-2 zugegeben. Nach beendetem Aufbau des Molgewichtes und Erreichen des gewünschten Viskositätsbereiches rührte man weitere 4,5 Stunden, um den restlichen Isocyanatgehalt mit iso-Propanol zu blockieren. Man erhielt 803 g einer 28,4%igen Polyurethanharnstofflösung in Toluol/iso-Propanol/1-Methoxypropanol-2 mit einer Viskosität von 3250 mPas bei 22 °C.

### Beispiel 20: (erfindungsgemäß)

97,8 g Desmophen C 2200, 48,9 g Polycarbonatdiol des Beispiels 2, 40,0 g LB 25 und 47,8 g 4,4'-Bis(isocyanatocyclohexyl)methan (H₁₂MDI) wurden bei 110 °C bis zu einem konstanten NCO-Gehalt von 2,7 % umgesetzt. Man ließ abkühlen und verdünnte mit 320,0 g Toluol und 200 g iso-Propanol. Bei Raumtemperatur wurden eine Lösung von 12,3 g Isophorondiamin in 100 g 1-Methoxypropanol-2 zugegeben. Nach beendetem Aufbau des Molgewichtes und Erreichen des gewünschten Viskositätsbereiches rührte man weitere 3,5 Stunden, um den restlichen Isocyanatgehalt mit iso-Propanol zu blockieren. Man erhielt 867 g einer 28,9%igen Polyurethanharnstofflösung in Toluol/iso-Propanol/1-Methoxypropanol-2 mit einer Viskosität von 6200 mPas bei 22 °C.

### Beispiel 21: Kontaktwinkel und 100%-Module von Vergleichsbeispiel 17 gegen erfinderische Beispiele 18, 19 und 20

Die Herstellung der Beschichtungen sowie die Ermittlung der Kontaktwinkel und 100%-Module erfolgt wie im Beispiel 12 beschrieben.

**Tabelle 2: Kontaktwinkel und 100%-Module der Filme aus Materialien der Beispiele 17, 18, 19 und 20**

| Beispiel-Nr. | Kontaktwinkel (°) | 100%-Modul (N/mm²) |
|---|---|---|
| Vergleichsbeispiel 17 | 14 | 2,7 |
| Beispiel 18 | 20 | 4,6 |
| Beispiel 19 | 21 | 6,0 |
| Beispiel 20 | 18 | 6,3 |

Die erfindungsgemäßen Beispiele 18, 19 und 20 enthalten im Vergleich zum Vergleichsbeispiel 17 Anteile eines erfindungswesentlichen Polycarbonatdiols. Die Oberfläche der Beschichtung ist nach wie vor sehr hydrophil, während sich das 100%-Modul in etwa verdreifacht.

## Patentansprüche

1. Polyurethanharnstofflösungen umfassend mindestens einen Polyurethanharnstoff, der Struktureinheiten der Formel (I) 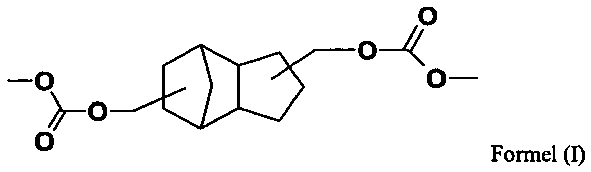 aufweist und mit wenigstens einer Copolymereinheit aus Polyethylenoxid und Polypropylenoxid terminiert ist.

2. Polyurethanharnstofflösungen gemäß Anspruch 1, **dadurch gekennzeichnet, dass** die darin enthaltenen Polyurethanharnstoffe frei von ionischen oder ionogenen Gruppen sind.

3. Polyurethanharnstofflösungen gemäß Anspruch 1 oder 2, **dadurch gekennzeichnet, dass** die darin enthaltenen Polyurethanharnstoffe auf einer Polycarbonatpolyolkomponente, basieren, die eine mittlere Hydroxylfunktionalität von bevorzugt 1,7 bis 2,3 aufweist.

4. Polyurethanharnstofflösungen gemäß Anspruch 3, **dadurch gekennzeichnet, dass** Polycarbonatpolyolkomponente Polycarbonatpolyole a1) aufweist, die durch Umsetzung von Kohlensäurederivaten mit difunktionellen Alkoholen der Formel (II) 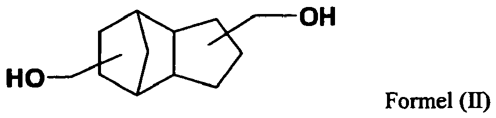 erhalten werden.

5. Polyurethanharnstofflösungen gemäß Anspruch 4, **dadurch gekennzeichnet, dass** Polycarbonatpolyolkomponente neben Polycarbonatpolyolen a1) auch weitere Polycarbonatpolyolen a2) aufweist.

6. Polyurethanharnstofflösungen gemäß Anspruch 5, **dadurch gekennzeichnet, dass** es sich bei den Polycarbonatpolyolen a2) um Verbindungen mit mittleren Hydroxylfunktionalitäten von 1,7 bis 2,3 und durch die OH-Zahl bestimmte Molekulargewichte von 400 bis 6000 g/mol auf Basis von Hexandiol-1,6, Butandiol-1,4 oder deren Mischungen handelt.

7. Polyurethanharnstofflösungen gemäß einem der Ansprüche 1 bis 6, **dadurch gekennzeichnet, dass** die zur Terminierung eingesetzte Copolymereinheit aus Polyethylenoxid und Polypropylenoxid auf einem monohydroxyfunktionellen gemischten Polyalkylenoxidpolyether aus mindestens 40 mol-% Ethylenoxid- und maximal 60 mol-% Propylenoxideinheiten bezogen auf den Gesamtanteil an Alkylenoxideinheiten basiert mit einem zahlenmittleren Molekulargewicht von 500 g/mol bis 5000 g/mol.

8. Polyurethanharnstofflösungen gemäß einem der Ansprüche 1 bis 7, **dadurch gekennzeichnet, dass** darin enthaltenen Polyurethanharnstoffe zahlenmittlere Molekulargewichte von 5000 bis 100000 g/mol gemessen in Dimethylacetamid bei 30 °C aufweisen.

9. Polyurethanharnstofflösungen gemäß einem der Ansprüche 1 bis 8, **dadurch gekennzeichnet, dass** diese als Lösungsmittel Dimethylformamid, N-Methylacetamid, Tetramethylharnstoff, N-Methylpyrrolidon, Toluol, lineare und cyclische Ester, Ether, Ketone, Alkohole oder deren Mischungen enthalten.

10. Polyurethanharnstofflösungen gemäß Anspruch 9, **dadurch gekennzeichnet, dass** diese als Lösungsmittel Mischungen aus Toluol und Ethanol, n-Propanol, iso-Propanol und/oder 1-Methoxy-2-propanol enthalten.

11. Polyurethanharnstofflösungen gemäß einem der Ansprüche 1 bis 10, **dadurch gekennzeichnet, dass** diese pharmakologische Wirkstoffe umfassen.

12. Verfahren zur Herstellung der Polyurethanharnstofflösungen gemäß einem der Ansprüche 1 bis 11, bei dem eine Polycarbonatpolyolkomponente a), mindestens eine Polyisocyanatkomponente b), mindestens eine Polyoxyalkylenetherkomponente c), mindestens eine Diamin- und/oder Aminoalkoholkomponente d) und gegebenenfalls eine weitere Polyolkomponente miteinander umgesetzt werden.

13. Polyurethanharnstoffe erhältlich aus Polyurethanharnstofflösungen gemäß einem der Ansprüche 1 bis 11.

14. Beschichtungen erhältlich unter Verwendung von Polyurethanharnstoffen gemäß Anspruch 13.

15. Substrate beschichtet mit Beschichtungen gemäß Anspruch 14.

## Claims

1. Polyurethaneurea solutions comprising at least one polyurethaneurea which has structural units of the formula (I) 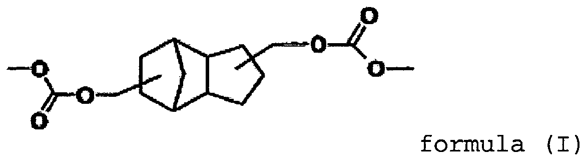 and is terminated with at least one copolymer unit of polyethylene oxide and polypropylene oxide.

2. Polyurethaneurea solutions according to Claim 1, **characterized in that** the polyurethaneureas present therein are free from ionic or ionogenic groups.

3. Polyurethaneurea solutions according to Claim 1 or 2, **characterized in that** the polyurethaneureas present therein are based on a polycarbonate polyol component, which has an average hydroxyl functionality of preferably 1.7 to 2.3.

4. Polyurethaneurea solutions according to Claim 3, **characterized in that** polycarbonate polyol component has polycarbonate polyols a1) which are obtained by reacting carbonic acid derivatives with difunctional alcohols of the formula (II)

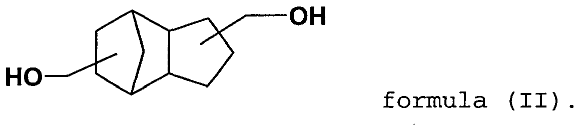

5. Polyurethaneurea solutions according to Claim 4, **characterized in that** polycarbonate polyol component has not only polycarbonate polyols a1) but also further polycarbonate polyols a2).

6. Polyurethaneurea solutions according to Claim 5, **characterized in that** the polycarbonate polyols a2) are compounds having average hydroxyl functionalities of 1.7 to 2.3 and molecular weights, as determined through the OH number, of 400 to 6000 g/mol, based on hexane-1,6-diol, butane-1,4-diol or mixtures thereof.

7. Polyurethaneurea solutions according to any of Claims 1 to 6, **characterized in that** the copolymer unit of polyethylene oxide and polypropylene oxide that is used for termination is based on a monohydroxy-functional mixed polyalkylene oxide polyether of at least 40 mol% ethylene oxide units and not more than 60 mol% propylene oxide units, based on the total fraction of alkylene oxide units, with a number-average molecular weight of 500 g/mol to 5000 g/mol.

8. Polyurethaneurea solutions according to any of Claims 1 to 7, **characterized in that** polyurethaneureas present therein have number-average molecular weights of 5000 to 100 000 g/mol as measured in dimethylacetamide at 30°C.

9. Polyurethaneurea solutions according to any of Claims 1 to 8, **characterized in that** they contain, as solvent, dimethylformamide, N-methylacetamide, tetramethylurea, N-methylpyrrolidone, toluene, linear and cyclic esters, ethers, ketones, alcohols or mixtures thereof.

10. Polyurethaneurea solutions according to Claim 9, **characterized in that** they contain, as solvent, mixtures of toluene and ethanol, n-propanol, isopropanol and/or 1-methoxy-2-propanol.

11. Polyurethaneurea solutions according to any of Claims 1 to 10, **characterized in that** they comprise pharmacological actives.

12. Process for preparing the polyurethaneurea solutions according to any of Claims 1 to 11, in which a polycarbonate polyol component a), at least one polyisocyanate component b), at least one polyoxyalkylene ether component c), at least one diamine and/or amino alcohol component d) and, if desired, a further polyol component are reacted with one another.

13. Polyurethaneureas obtainable from polyurethaneurea solutions according to any of Claims 1 to 11.

14. Coatings obtainable using polyurethaneureas according to Claim 13.

15. Substrates coated with coatings according to Claim 14.

## Revendications

1. Solutions de polyuréthane-urées comprenant au moins une polyuréthane-urée qui comporte des motifs structuraux de formule (I) 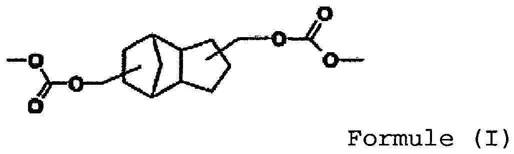 et est terminée par au moins une unité copolymère de polyoxyéthylène et polyoxypropylène.

2. Solutions de polyuréthane-urées selon la revendication 1, **caractérisées en ce que** les polyuréthane-urées contenues dans celles-ci sont exemptes de groupes ioniques ou ionogènes.

3. Solutions de polyuréthane-urées selon la revendication 1 ou 2, **caractérisées en ce que** les polyuréthane-urées contenues dans celles-ci sont à base d'un composant polycarbonatepolyol qui présente une fonctionnalité hydroxy moyenne de préférence de 1,7 à 2,3.

4. Solutions de polyuréthane-urées selon la revendication 3, **caractérisées en ce que** le composant polycarbonatepolyol comporte des polycarbonatepolyols a1) qui sont obtenus par la réaction de dérivés d'acide carbonique avec des alcools difonctionnels de formule (II)

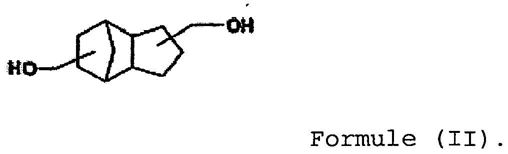

5. Solutions de polyuréthane-urées selon la revendication 4, **caractérisées en ce que** le composant polycarbonatepolyol comporte, outre des polycarbonatepolyols a1), également d'autres polycarbonatepolyols a2).

6. Solutions de polyuréthane-urées selon la revendication 5, **caractérisées en ce que** les polycarbonatepolyols a2) consistent en des composés ayant des fonctionnalités hydroxy moyennes de 1,7 à 2,3 et des masses moléculaires, déterminées par l'indice de groupes OH, de 400 à 6 000 g/mole, à base d'hexanediol-1,6, de butanediol-1,4 ou de mélanges de ceux-ci.

7. Solutions de polyuréthane-urées selon l'une quelconque des revendications 1 à 6, **caractérisées en ce que** l'unité copolymère de polyoxyéthylène et polyoxypropylène, utilisée pour la terminaison, est à base d'un polyoxyalkylène-polyéther mixte à fonctionnalité monohydroxy, composé d'au moins 40 % en moles de motifs oxyde d'éthylène et d'au maximum 60 % en moles de motifs oxyde de propylène, par rapport à la quantité totale de motifs oxyde d'alkylène, ayant une masse moléculaire moyenne en nombre de 500 g/mole à 5 000 g/mole.

8. Solutions de polyuréthane-urées selon l'une quelconque des revendications 1 à 7, **caractérisées en ce que** les polyuréthane-urées contenues dans celles-ci ont des masses moléculaires moyennes en nombre de 5 000 à 100 000 g/mole, mesurées dans du diméthylacétamide à 30°C.

9. Solutions de polyuréthane-urées selon l'une quelconque des revendications 1 à 8, **caractérisées en ce qu'**elles contiennent comme solvant du diméthylformamide, du N-méthylacétamide, de la tétraméthylurée de la N-méthylpyrrolidone, du toluène, des esters, éthers, cétones, alcools, linéaires et cycliques, ou des mélanges de ceux-ci.

10. Solutions de polyuréthane-urées selon la revendication 9, **caractérisées en ce qu'**elles contiennent comme solvant des mélanges de toluène et éthanol, n-propanol, isopropanol et/ou 1-méthoxy-2-propanol.

11. Solutions de polyuréthane-urées selon l'une quelconque des revendications 1 à 10, **caractérisées en ce qu'**elles comprennent des substances actives pharmacologiques.

12. Procédé pour la préparation des solutions de polyuréthane-urées selon l'une quelconque des revendications 1 à 11, dans lequel on fait réagir entre eux un composant polycarbonatepolyol a), au moins un composant polyisocyanate b), au moins un composant polyoxyalkylène-éther c), au moins un composant diamine et/ou aminoalcool d) et éventuellement un autre composant polyol.

13. Polyuréthane-urées pouvant être obtenues à partir de solutions de polyuréthane-urées selon l'une quelconque des revendications 1 à 11.

14. Revêtements pouvant être obtenus avec utilisation de polyuréthane-urées selon la revendication 13.

15. Substrats revêtus avec des revêtements selon la revendication 14.
